Europäisches Patentamt

European Patent Office

Office européen des brevets

(19)

(11) Veröffentlichungsnummer : **0 169 396**
**B1**

(12) **EUROPÄISCHE PATENTSCHRIFT**

(45) Veröffentlichungstag der Patentschrift :
21.01.87

(21) Anmeldenummer : 85107884.0

(22) Anmeldetag : 25.06.85

(51) Int. Cl.⁴ : **C 07 C 31/20, C 07 C 29/136**

(54) **Verfahren zur Herstellung von 1,4-Butandiol.**

(30) Priorität : 26.06.84 DE 3423447

(43) Veröffentlichungstag der Anmeldung :
29.01.86 Patentblatt 86/05

(45) Bekanntmachung des Hinweises auf die Patenterteilung : 21.01.87 Patentblatt 87/04

(84) Benannte Vertragsstaaten :
BE CH DE FR GB IT LI NL

(56) Entgegenhaltungen :
EP-A- 0 058 940
EP-A- 0 060 787
DE-A- 2 543 673
DE-B- 2 845 905
US-A- 4 032 458
US-A- 4 172 961

(73) Patentinhaber : BASF Aktiengesellschaft
Carl-Bosch-Strasse 38
D-6700 Ludwigshafen (DE)

(72) Erfinder : Schnabel, Rolf, Dr.
Frankenstrasse 22
D-6707 Schifferstadt (DE)
Erfinder : Weitz, Hans-Martin, Dr.
Auf dem Koeppel 40
D-6702 Bad Duerkheim (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die vorliegende Erfindung betrifft ein Verfahren zur Herstellung von 1,4-Butandiol durch katalytische Hydrierung von Bernsteinsäuredibutylester.

Bekanntlich läßt sich 1,4-Butandiol durch hydrogenolytische Spaltung von Bernsteinsäuredibutylester herstellen. Bei diesem, z. B. in der DE-OS-25 43 673 beschriebenen Verfahren hydriert man den Bernsteinsäuredibutylester in Gegenwart eines Kupferchromitkatalysators unter einem Wasserstoffdruck von 100 bis 300 bar und bei Temperaturen von 200 bis 260 °C. Aus dem dabei erhaltenen Gemisch von 1,4-Butandiol und Butanol trennt man das Diol ab, während man das Butanol erneut für die Veresterung des als Ausgangsverbindung verwendeten Maleinsäureanhydrids heranzieht. Nach einem ähnlichen, in der US-PS-4 032 458 beschriebenen Verfahren, destilliert man das Butanol aus dem Hydriergemisch ab. Zum Rückstand, der das rohe 1,4-Butandiol enthält, gibt man Wasser, extrahiert aus dem wäßrigen 1,4-Butandiol Verunreinigungen durch Behandlung mit flüssigen Kohlenwasserstoffen und gewinnt das reine 1,4-Butandiol durch Destillation der so behandelten wäßrigen Lösung.

Wie sich bei der katalytischen Hydrierung des Bernsteinsäuredibutylesters gezeigt hat, sind die Reaktionsgeschwindigkeiten häufig so niedrig, daß pro Raum- und Zeiteinheit nur relativ wenig 1,4-Butandiol gebildet wird. Dieser Mangel bedeutet für eine technische Anwendung, daß große und entsprechend aufwendige Anlagen sowie erhebliche Katalysatormengen benötigt werden. Es bestand somit die Aufgabe, die Raum-Zeit-Ausbeuten bei dem genannten Verfahren zu verbessern.

Es wurde nun gefunden, daß man bei der Herstellung von 1,4-Butandiol durch katalytische Hydrierung von Bernsteinsäuredibutylester bei Temperaturen von 150 bis 300 °C und Drücken von über 100 bar, besonders vorteilhafte Ergebnisse und insbesondere höhere Raum-Zeit-Ausbeuten erhält, wenn man mit nicht vollständigem Umsatz hydriert, aus dem Reaktionsgemisch Butanol abdestilliert, den flüssigen Destillationsrückstand auf Temperaturen unter 108 °C abkühlt, die dabei durch Phasentrennung entstandene obere Phase, die im wesentlichen aus Bernsteinsäuredibutylester besteht, in die Hydrierung zurückleitet und aus der unteren Phase, die im wesentlichen aus 1,4-Butandiol besteht, das 1,4-Butandiol durch fraktionierte Destillation im Vakuum gewinnt.

Nach dem neuen Verfahren hydriert man den Bernsteinsäuredibutylester auf an sich bekannte Weise in Gegenwart von Hydrierungskatalysatoren bei Temperaturen von 150 bis 300 °C, insbesondere 170 bis 190 °C, und bei Drücken von über 100 bar, insbesondere 200 bis 300 bar, wobei man bevorzugt kupferhaltige Katalysatoren verwendet und die Hydrierung kontinuierlich entsprechend der an sich üblichen Kreislauffahrweise (siehe Beispiel) durchführt. Erfindungsgemäß wird mit nicht vollständigem Umsatz hydriert, d. h. man hydriert nur bis zu einem Umsatz von maximal 97 %, vorzugsweise bis zu einem Umsatz von maximal 90 %. Die dabei erhältlichen Reaktionsgemische liegen in ihrer Zusammensetzung zwischen den Konzentrationsbereichen :

91,7 bis 2,9 Gew.% Bernsteinsäuredibutylester
5,2 bis 60,4 Gew.% Butanol
3,1 bis 36,7 Gew.% 1,4-Butandiol,

vorzugsweise

49,1 bis 9,7 Gew.% Bernsteinsäuredibutylester,
31,6 bis 56,2 Gew.% Butanol
19,2 bis 34,1 Gew.% 1,4-Butandiol.

Aus diesen Reaktionsgemischen destilliert man Butanol ab. Man destilliert zweckmäßigerweise bei Normaldruck oder leichtem Vakuum in einer Kolonne bei Sumpftemperaturen von z. B. 125 bis 220 °C und Kopftemperaturen von 108 bis 120 °C, wobei man ein Destillat erhält, das Butanol als Hauptkomponente enthält. Der Butanolgehalt im flüssigen Sumpfrückstand sollte dabei auf Werte unter 3 Gew.% zurückgehen. Der flüssige Destillationsrückstand wird dann auf Temperaturen unter 108 °C abgekühlt, wobei Phasentrennung eintritt. Es bilden sich zwei flüssige Phasen, von denen die obere Phase im wesentlichen aus dem nicht umgesetzten Bernsteinsäuredibutylester und die untere Phase im wesentlichen aus 1,4-Butandiol besteht. Die obere Phase leitet man in die Hydrierung zurück, wobei man sie vorteilhaft als Kühlmedium für den Kreislaufstrom verwendet. Aus der unteren Phase gewinnt man das 1,4-Butandiol durch fraktionierte Destillation im Vakuum. Man destilliert beispielsweise in einer Kolonne bei einem Druck von 20 bis 26 mbar, bei Sumpftemperaturen von 146 bis 152 °C und Kopftemperaturen von 100 bis 127 °C. Man erhält dabei einen zweiphasigen Vorlauf, der praktisch den gesamten Bernsteinsäuredibutylester enthält und den man zweckmäßigerweise zum oben beschriebenen Phasenabscheider zurückführt, während man als Hauptlauf das 1,4-Butandiol gewinnt.

Nach dem erfindungsgemäßen Verfahren erhält man 1,4-Butandiol aus Bernsteinsäuredibutylester auf besonders vorteilhafte Weise und mit hoher Raum-Zeit-Ausbeute. Überraschenderweise erfordert das neue Verfahren einen relativ geringen Aufwand für die Abtrennung des 1,4-Butandiols aus dem Reaktionsgemisch, denn bei einer Arbeitsweise, bei der ein nicht vollständiger Umsatz vorausgesetzt wird, hätte man mit einem zusätzlichen Destillationsaufwand zur Auftrennung der Reaktionsgemische rechnen müssen. Der tatsächliche Destillationsaufwand ist aber unerwartet gering, wobei sich als besonders überraschend herausgestellt hat, daß man als Vorlauf einer einfachen Vakuumdestillation Reste an Bernsteinsäuredibutylester mit Butandiol als Hetero-Azeotrop vom 1,4-Butandiol abtrennen kann.

Beispiel (siehe Figur)

Es werden die folgenden Abkürzungen verwendet :

BSBE = Bernsteinsäuredibutylester
BuOH = Butanol
BA = 1,4-Butandiol

In einem Reaktor (1) mit den Abmessungen 111 × 8 000 mm wurden in einem kontinuierlichen Versuch 8 t BSBE bei 180 °C und einem Wasserstoffdruck von 250 bar hydriert. Der Reaktor enthielt 51 l eines Festbett-Katalysators, der aus 60 Gew.% Cu und 40 Gew.% $SiO_2$ bestand und der in 5 × 5 mm-Tablettenform vorlag. Der Reaktor war eingebunden in einen Flüssigkeits- und Gaskreislauf (2), in dem 350 l/h Flüssigkeit und 60 $Nm^3$/h Wasserstoff unter 250 bar strömten. Dem Flüssigkeitskreislauf wurden 45 l/h BSBE zugeführt (3), die entsprechende Menge flüssigen Reaktionsgemisches wurde ihm entnommen (4). Der abreagierte Wasserstoff wurde über eine Druckregelung laufend ergänzt (5). Die Temperatur am Reaktoreingang (oben) betrug 163 °C, am Reaktorausgang (unten) 182 °C. Die GC-Analyse des flüssigen Reaktionsgemisches, das dem Reaktor entnommen wurde, ergab 30 Gew.% BA, 54 Gew.% BuOH und 14 Gew.% BSBE. Der Umsatz betrug somit 86 % und die Raum-Zeit-Ausbeute 0,26 kg BA/l · h.

Von dem flüssigen Reaktionsgemisch, das dem Reaktor entnommen wurde, wurden 1,6 kg in eine Destillationsapparatur (6) mit einer 10 cm-Vigreux-Kolonne und einer Destillationsbrücke mit Liebig-Kühler geleitet. Unter atmosphärischem Druck wurden bei Sumpftemperaturen von 125 bis 216 °C und Kopftemperaturen von 108 bis 118 °C 0,87 kg Destillat (7) gewonnen, das BuOH als Hauptkomponente und nach der GC-Analyse < 0,1 Gew.% BA und 0,1 Gew.% BSBE enthielt.

Der flüssige Sumpfrückstand wurde in einem Abscheider (8) auf 25 °C abgekühlt, wobei Phasentrennung eintrat. Die obere Phase (9) (0,19 kg) enthielt nach der GC-Analyse 1,2 Gew.% BuOH, 3,5 Gew.% BA und 91 Gew.% BSBE. Sie wurde in die Hydrierung zurückgeleitet (10). Die untere Phase (11) (0,54 kg) enthielt nach der GC-Analyse 2,7 Gew.% BuOH, 83 Gew.% BA und 8,0 Gew.% BSBE.

Die untere Phase wurde abgetrennt und in der Kolonne (12) destilliert. Bei einem Druck von 20 bis 26 mbar und bei Sumpftemperaturen von 146 bis 152 °C und Kopftemperaturen von 100 bis 127 °C fiel ein zweiphasiger Vorlauf (13) von 0,15 kg an, der praktisch den gesamten BSBE enthielt. Der Hauptlauf (14), der bei 20 mbar, Sumpftemperaturen von 152 bis 225 °C und Kopftemperaturen von 127 bis 128 °C gewonnen wurde und der im wesentlichen aus BA bestand, enthielt weniger als 0,2 Gew.% BSBE.

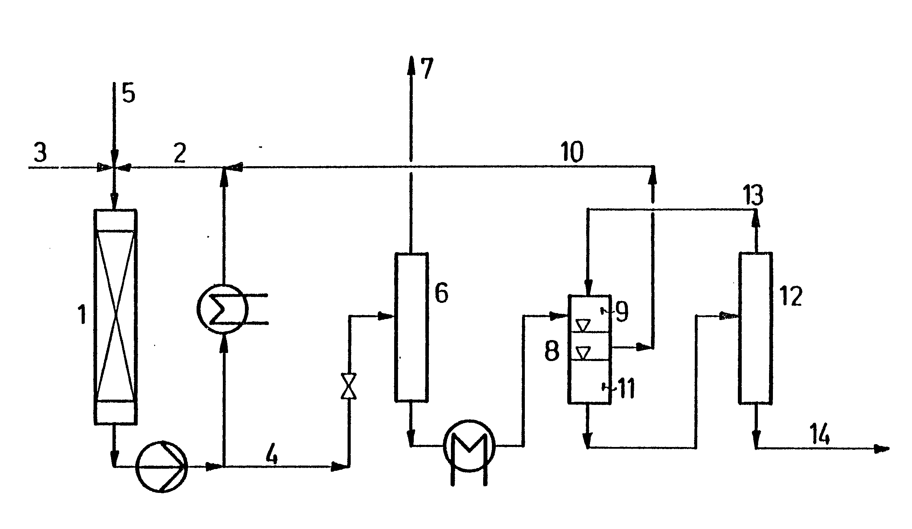

**Patentansprüche**

1. Verfahren zur Herstellung von 1,4-Butandiol durch katalytische Hydrierung von Bernsteinsäuredibutylester bei Temperaturen von 150 bis 300 °C und Drücken von über 100 bar, dadurch gekennzeichnet, daß man den Bernsteinsäuredibutylester nur bis zu einem Umsatz von maximal 97 % hydriert, aus dem Reaktionsgemisch Butanol abdestilliert, den flüssigen Destillationsrückstand auf Temperaturen unter 108 °C abkühlt, die dabei durch Phasentrennung entstandene obere Phase, die im wesentlichen aus Bernsteinsäuredibutylester besteht, in die Hydrierung zurückleitet und aus der unteren Phase, die im wesentlichen aus 1,4-Butandiol besteht, das 1,4-Butandiol durch fraktionierte Destillation im Vakuum gewinnt.

2. Verfahren nach Anspruch 1, dadurch gekennzeichnet, daß man den Bernsteinsäuredibutylester nur bis zu einem Umsatz von maximal 90 % hydriert.

3. Verfahren nach Anspruch 1, dadurch gekennzeichnet, daß man den Bernsteinsäuredibutylester an einem Kupfer enthaltenden Katalysator bei Temperaturen von 170 bis 190 °C und Drücken von 200 bis 300 bar hydriert.

4. Verfahren nach Anspruch 1, dadurch gekennzeichnet, daß man Butanol aus dem Reaktionsgemisch in einer Kolonne bei Sumpftemperaturen von 125 bis 220 °C und Kopftemperaturen von 108 bis 120 °C abdestilliert.

5. Verfahren nach Anspruch 1, dadurch gekennzeichnet, daß man aus der durch Phasentrennung erhaltenen unteren Phase durch fraktionierte Destillation in einer Kolonne bei einem Druck von 20 bis 26 mbar, bei Sumpftemperaturen von 146 bis 152 °C und Kopftemperaturen von 100 bis 127 °C das 1,4-Butanol als Hauptlauf gewinnt.

**Claims**

1. A process for the preparation of butane-1,4-diol by catalytic hydrogenation of dibutyl succinate at from 150 to 300 °C and under a pressure of above 100 bar, wherein the dibutyl succinate is hydrogenated only until the conversion reaches a maximum of 97 %, butanol is distilled off from the reaction mixture, the liquid distillation residue is cooled to below 108 °C, the upper phase, which is formed as a result of phase separation and consists essentially of dibutyl succinate, is recycled to the hydrogenation, and the butane-1,4-diol is obtained from the lower phase, which consists essentially of this compound, by fractional distillation under reduced pressure.

2. A process as claimed in claim 1, wherein the dibutyl succinate is hydrogenated only until the conversion reaches a maximum of 90 %.

3. A process as claimed in claim 1, wherein the dibutyl succinate is hydrogenated over a copper-containing catalyst at from 170 to 190 °C and under from 200 to 300 bar.

4. A process as claimed in claim 1, wherein butanol is distilled off from the reaction mixture in a column at a bottom temperature of from 125 to 220 °C and a top temperature of from 108 to 120 °C.

5. A process as claimed in claim 1, wherein the butane-1,4-diol is obtained as the main fraction by subjecting the lower phase, obtained as a result of phase separation, to fractional distillation in a column under a pressure of from 20 to 26 mbar and at a bottom temperature of from 146 to 152 °C and a top temperature of from 100 to 127 °C.

## Revendications

1. Procédé pour la préparation de 1,4-butanediol par hydrogénation catalytique d'ester dibutylique d'acide succinique à des températures de 150 à 300 °C et sous des pressions de plus de 100 bars, caractérisé en ce qu'on n'hydrogène l'ester dibutylique d'acide succinique que jusqu'à un degré de transformation de 97 % au maximum, on chasse le butanol du mélange réactionnel par distillation, on refroidit à des températures inférieures à 108 °C le résidu liquide de la distillation, on renvoie à l'hydrogénation la phase supérieure qui se forme dans ces conditions par séparation de phases et qui se compose essentiellement d'ester dibutylique d'acide succinique et on tire le 1,4-butanediol, par distillation fractionnée dans le vide, de la phase inférieure qui se compose essentiellement de 1,4-butanediol.

2. Procédé selon la revendication 1, caractérisé en ce qu'on hydrogène l'ester dibutylique d'acide succinique que jusqu'à un degré de transformation de 90 % au maximum.

3. Procédé selon la revendication 1, caractérisé en ce qu'on hydrogène l'ester dibutylique d'acide succinique en présence d'un catalyseur contenant du cuivre, à des températures de 170 à 190 °C et sous des pressions de 200 à 300 bars.

4. Procédé selon la revendication 1, caractérisé en ce qu'on chasse le butanol du mélange réactionnel par distillation dans une colonne à des températures de bas de colonne de 125 à 220 °C et des températures en tête de 108 à 120 °C.

5. Procédé selon la revendication 1, caractérisé en ce qu'à partir de la phase inférieure obtenue par séparation de phases, on tire le 1,4-butanediol en tant que produit de cœur, par distillation fractionnée dans une colonne sous une pression de 20 à 26 mbars, à des températures de bas de colonne de 146 à 152 °C et des températures en tête de 100 à 127 °C.

0 169 396